# EUROPEAN PATENT SPECIFICATION

(11) **EP 1 898 786 B1**
(45) Date of publication and mention of the grant of the patent: **30.03.2011**
(21) Application number: 06744066.9
(22) Date of filing: 02.06.2006
(51) Int. Cl.: A61B 5/08

(54) **A METHOD FOR GENERATING OUTPUT DATA**
VERFAHREN ZUR ERZEUGUNG VON AUSGABEDATEN
PROCEDE DE PRODUCTION DE DONNEES DE SORTIE

(30) Priority: 03.06.2005 GB 0511307; 07.06.2005 US 146905
(43) Date of publication of application: 19.03.2008
(73) Proprietor: South Manchester University Hospitals NHS Trust, Manchester M23 9LT (GB)
(72) Inventor: WOODCOCK, Ashley Arthur, Hale, Cheshire WA15 0LN (GB); SMITH, Jaclyn Ann, Manchester M16 0DR (GB); MCGUINNES, Kevin, Crewe, Cheshire CW3 0ES (GB)
(74) Representative: Kenrick, Mark Lloyd
(86) International application number: PCT/GB2006/002012
(87) International publication number: WO 2006/129098

(56) References cited:
- WO-A-2004/091503
- US-A- 5 928 156
- US-B1- 6 241 683
- US-B1- 6 261 238

## Description

The present invention relates to a method for processing data representing an acoustic signal to generate output data that identifies a class of a predetermined plurality of classes.

Coughing is one of the most common symptoms treated by medical practitioners and is often used to diagnose conditions such as asthma. Coughing is also a condition which is often painful and distressing for sufferers.

An assessment of a cough is typically subjective, being made by a medical practitioner during a consultation. There is no easy way to classify the severity of a cough in such a consultation. Instead, a medical practitioner must use their experience to subjectively assess the severity, and the likely cause of the cough.

One objective indication of the severity of a cough is to count the number of times a patient coughs over a period of time. Such an objective measure can be derived by monitoring a patient over a predetermined time period, and manually recording the number of coughs occurring within that time period. Although such monitoring may provide useful data, it is very labour intensive, requiring constant monitoring. Such monitoring is also intrusive to the patient.

Recently, attempts have been made to automatically and unobtrusively monitor cough events using an appropriately configured electronic device. However, such a device must distinguish cough events from environmental noises, and from speech of the subject. If such a device records a small percentage of speech as a cough, then the device will produce unreliable results, given that the time spent speaking is an order of magnitude greater than the time spent coughing.

PCT patent application publication number WO 2004/091503 discloses a method for estimating the lung volume of a subject. The method comprises processing signals indicative of a monitored subject's respiration, signals representative of the subject's posture and movement and signals representative of the sound made by the subject. The method also uses the rib cage size (RC) and the abdominal size (AB) of the subject while the subject undertakes different activities. From these measurements, the method determines an estimate of the lung volume (Vt) of the subject. The signals are then analysed to identic and distinguish between coughs, apnoea, hypopnoea, sighs and dyspnoea.

Although the method and apparatus described in WO 2004/091503 provides useful information, the number of sensors, and particularly the sensors worn to monitor the subject's abdomen and rib cage measurements cause such a device to be extremely uncomfortable. This is particularly undesirable given that a subject may be wearing the device for a substantial period of time.

It is an object of an embodiment of the present invention to obviate or mitigate at least some of the disadvantages set out above.

In this specification the term cough is used to mean as event in which a subject takes a breath in, followed by one or more forced expiratory efforts initially against a closed glottis, accompanied by a characteristic sound.

In accordance with an aspect of the present invention there is provided a calibration method for calibrating an apparatus to differentiate acoustic signals attributable to cough from other acoustic signals, the method comprising: receiving first data representing a first acoustic signal representing at least one cough; receiving second data representing a second acoustic signal representing an acoustic event other than a cough; and processing said first and second data to generate configuration data, for said apparatus, said configuration data defining at least one rule which when applied differentiates acoustic signals attributable to cough from other acoustic signals.

The inventors of the present invention have recognized that by calibrating an apparatus using a cough sample and a non cough sample, a much improved apparatus for differentiating between such acoustic signals can be created.

Output data may be generated identifying a class of a predetermined plurality of classes, by receiving data representing an acoustic signal; determining an amplitude of at least a first predetermined frequency component of said acoustic signal; comparing the or each amplitude with a respective primary threshold; and generating output data identifying one of said classes to which said acoustic signal should be allocated, based upon said comparison; wherein, the or each primary threshold is generated from first data representing an acoustic signal representing a first predetermined event, and second data representing an acoustic signal representing a second predetermined event.

By considering the amplitude of a frequency component of an acoustic signal, data, which can be used to classify acoustic signals more reliably, can be generated.

Apparatus may generate output data identifying a class of a predetermined plurality of classes. The apparatus comprises means for receiving data representing an acoustic signal; means for determining an amplitude of at least one first predetermined frequency component of said acoustic signal; means for comparing the or each amplitude with a respective primary threshold; and means for generating output data identifying one of said classes to which said acoustic signal should be allocated, based upon said comparison; wherein, the or each primary threshold is generated from first data representing an acoustic signal representing a first predetermined event, and second data representing an acoustic signal representing a second predetermined event.

Apparatus to output data identifying a class of a predetermined plurality of classes, each class representing a condition of a human or animal subject, may be callibrated by receiving data representing a first acoustic signal, said first acoustic signal being obtained at a first predetermined volume of air within the subjects lungs; and calibrating said apparatus based upon said acoustic signal.

The inventors of the present invention have also recognized that to calibrate such an apparatus, the quantity of air within the lungs of the subject is of extreme importance while producing calibration data, if a reliable calibration is to occur.

Output data identifying a class of a predetermined plurality of classes may be generated by receiving data representing an acoustic signal; determining the integral of the acoustic signal; comparing tube integral of the acoustic signal with a primary threshold; and generating output data identifying one of said classes to which said acoustic signal should be allocated, based upon said comparison; wherein, the predetermined frequency threshold and primary threshold are generated from first data representing an acoustic signal representing a first predetermined event, and second data representing an acoustic signal representing a second predetermined event.

By determining the integral of an acoustic signal, data, which can be used to classify acoustic signals more reliably, can be generated.

Output data indicative of a volume of air within a human or animal subject's lungs when the subject coughs, maybe generated by a method comprising: receiving data representing an acoustic signal representing a cough event; determining the integral of the acoustic signal; comparing the integral of the acoustic signal with calibration data; and generating output data indicative of the volume of air within the subject's lungs at which the subject coughs, based upon said comparison.

By determining the integral of an acoustic signal, data, which can be used to provide clinical information relating to the lung volume from which a patient coughed, can be generated.

Embodiments of the present invention will now be described, by way of example only, with reference to the accompanying drawings, in which:
Figure 1 is a flowchart showing an overview of operation of an embodiment of the present invention;
Figure 2 is a schematic illustration showing sensor placement on a subject's body, for use in the embodiment of Figure 1;
Figure 3 is a schematic illustration of the architecture of apparatus used in the embodiment of Figure 1;
Figure 4 is a graph showing amplitudes of various frequency components of vibrations caused by coughs, when different quantities of air are present in a subject's lungs;
Figure 5 is a flowchart of calibration carried out in Figure 1 in further detail;
Figure 6 is a flowchart of a process for determination of a subject's vital capacity;
Figure 7 is a graph representing air movement against time in the process of Figure 6;
Figure 8 is a flowchart showing a process for determining the quantity of air to be present within a subject's lungs, in the process of Figure 1;
Figure 9 is an illustration of a visual indicator showing an extent to which a subject should breathe out;
Figure 10 is a graph representing the amplitude of vibrations caused by a cough against time;
Figure 11 is a flow chart of a filtration process used to remove data which is not relevant to cough determination;
Figure 12 is a flowchart of a process used to remove outlying signals;
Figures 13a and 13b are graphs showing the effect of removing outlying signals from obtained data;
Figure 14 is a graph showing the location of threshold values derived during the calibration process;
Figure 15 is a flowchart showing the distinguishing step of Figure 1 in further detail;
Figure 16 is a flowchart showing the method of determining an area under an amplitude / time curve in accordance with a further embodiment of the present invention; and
Figure 17 is a graph showing the area under an amplitude / time curve for an acoustic signal relating to a cough event in accordance with a the embodiment of Figure 16.

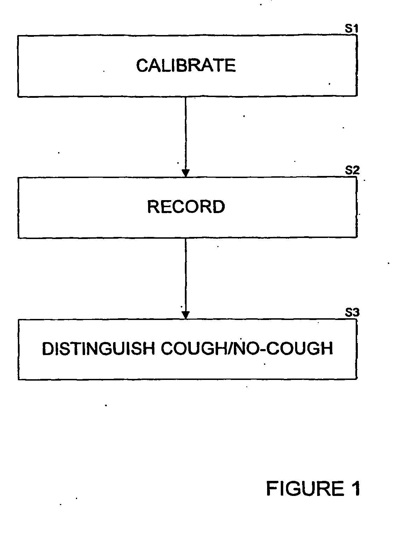

Referring first to Figure 1, there is illustrated an overview of processing carried out in an embodiment of the present invention, to distinguish data indicative of a subject coughing from data indicative of other sounds. A first step S1 involves calibration of apparatus, a second step S2 involves recording data representing an acoustic signal, and a third step S3 involves processing data obtained at step S2 to distinguish data indicative of cough from data indicative of other events.

The calibration of step S1, the recording of step S2 and the distinguishing of step S3 all involve processing acoustic signals obtained from a subject. Such acoustic signals are obtained using a sensor positioned on the subject's chest. It will be appreciated that much of the processing of step S3 will distinguish acoustic data attributable to cough from acoustic data attributable to speech. It has been realised that vibrations at the chest wall differ considerably depending on whether the vibrations were caused by cough or by speech, thus sensor placement on the subject's chest allows acoustic signals attributable to cough to be distinguished from those attributable to speech more easily than, for example, using an air-coupled or lapel microphone.

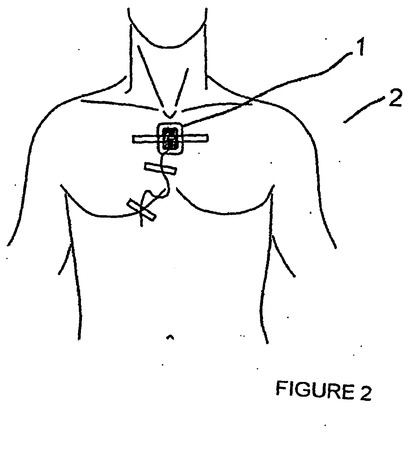

Figure 2 illustrates a piezoelectric sensor 1 positioned on a subject's chest 2, the piezoelectric sensor being configured to convert vibrations of the subject's chest wall indicative of an acoustic signal generated by the subject, into an electric signal. It should be noted that the piezoelectric sensor 1 does not detect acoustic signals attributable to other sources. As illustrated in Figure 2, the piezoelectric sensor 1 is attached to the subject on the manubrium of the subject's sternum, directly below the suprasternal notch. The piezoelectric sensor 1 is positioned was shown in Figure 2 as it has been found that such positioning keeps the sensor 1 substantially stable, even if the subject moves. Additionally the illustrated position is such as to be the part of the subject's chest where there is least tissue between the skin and the airway.

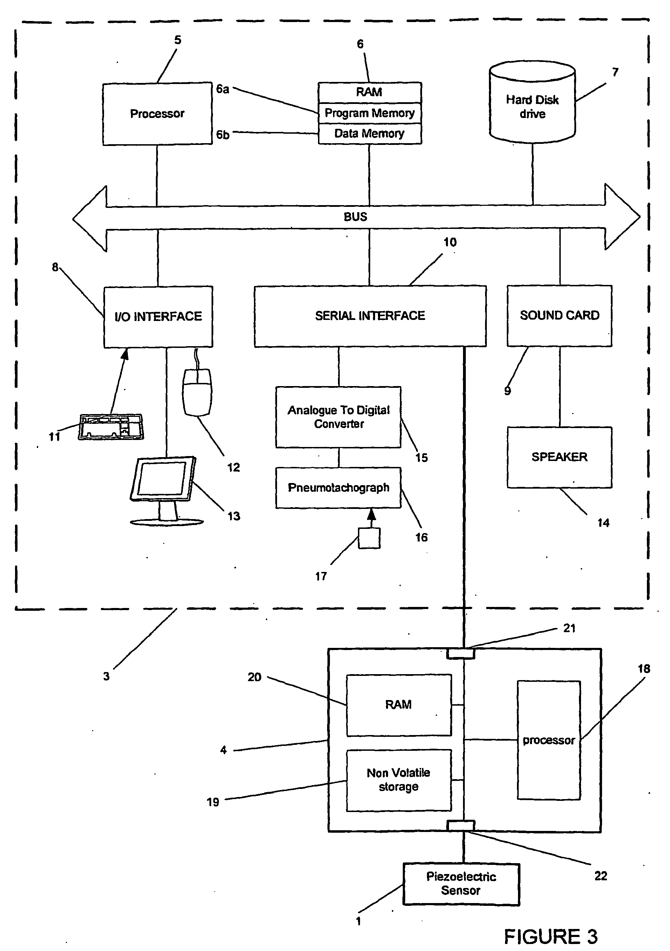

Having described the use and positioning of the piezoelectric sensor 1, apparatus used in embodiments of the present invention is now described in further detail with reference to Figure 3. The illustrated apparatus comprises three main components, the piezoelectric sensor 1 described above, a workstation 3, and a sensor interface 4 configured to receive data from the piezoelectric sensor 1 and provide this data to the workstation 3. It can be seen that the workstation 3 comprises a processor 5 and Random Access Memory (RAM) 6. The RAM 6 is, in use, divided to provide a program memory 6a and a data memory 6b. The processor 5 is configured to execute instructions specified in programs stored in the program memory 6b. The workstation 3 further comprises a non-volatile storage device in the form of a hard disk drive 7. The workstation 3 also comprises an input/output (I/O) interface 8, a sound card 9 and a serial interface 10. The processor 5, the RAM 6, the hard disk drive 7, the I/O interface 8, the sound card 9 and the serial interface 10 are connected together by means of a bus along which both data and instructions are passed.

The I/O interface 8 controls the flow of data between input and output devices and other components of the workstation 3. It can be seen that the I/O interface is connected to a keyboard 11, a mouse 12 and a display screen 13. The sound card 9 is configured to handle output of audio data, and it can be seen that the sound card 9 is connected to a speaker 14 to which audio data is passed. The serial interface 10 allows the workstation 3 to be coupled to various external devices, and it can be seen that the sensor interface 4 mentioned above is connected to the workstation 3 by means of the serial interface 10. The serial interface 10 is further connected to an analogue to digital converter 15 which in turn is connected to a pneumotachograph 16. It will be appreciated that other devices capable of measuring lung volumes can be used.

The pneumotachograph 16 is a conventional piece of apparatus for measuring the amount of air exhaled and inhaled by a subject, and its use is described below. A spirometer could be used in place of a pneumotachograph in some embodiments of the invention. It can be seen from Figure 3, that the pneumotachograph 16 is connected to a mouthpiece 17.

The piezoelectric sensor 1 communicates electrical signals to the workstation 3 via the sensor interface 4. The sensor interface 4 comprises a processor 18, non-volatile storage 19 and RAM 20. The sensor interface 4 further comprises a connector 21 for connection to the workstation 3 and an input 22 for connection to the piezoelectric sensor 1. It should be noted that acoustic signals represented by electrical signals provided by the piezoelectric sensor 1, may be stored in the non-volatile storage 19, thereby allowing the sensor interface 4 to be used as a standalone device for the collection of data, without requiring connection to the workstation 3.

Having described the architecture of apparatus used to implement the present invention, calibration of the apparatus (step S1 of Figure 1) is now described.

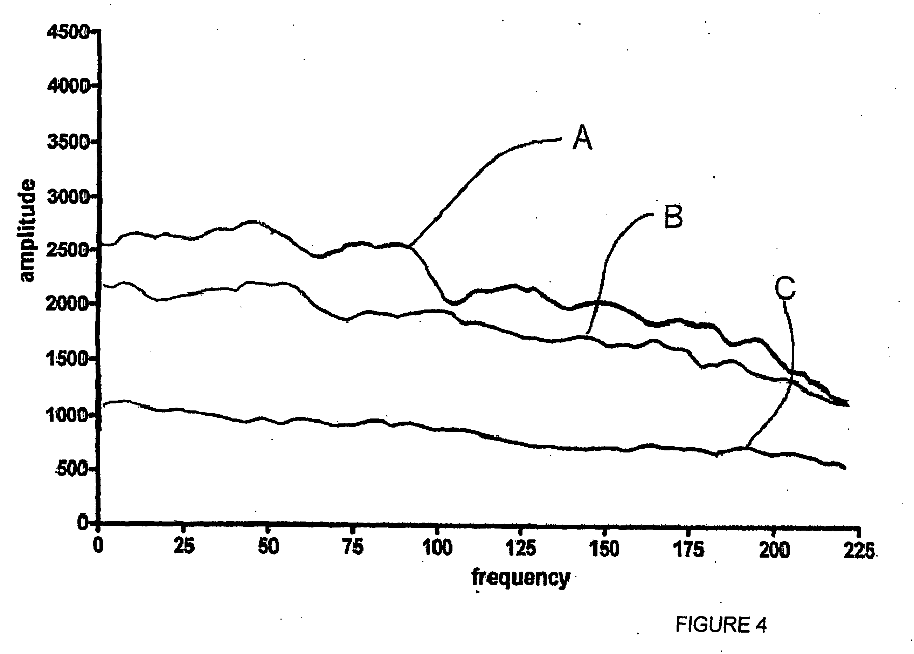

Experiments have shown that the quantity of air within a subject's lungs when a cough occurs, is a major source of variability in vibrations caused by a cough at the chest wall. Vital capacity is a term used hereinafter to refer to the capacity of a subject's lungs, and Figure 4 shows plots of the amplitudes of vibrations of various frequencies, caused by coughs produced by a subject when the quantity of air in the subject's lungs was 90%, 60% and 30% of their vital capacities (lines A, B, C respectively). It can be seen from Figure 4 that, the greater the quantity of air within a subject's lungs (expressed as a fraction of vital capacity) the greater the amplitudes of the generated vibrations.

It should be noted that the amplitudes of vibrations of the chest wall caused by speech are typically small as compared with vibrations caused by cough. Thus, by carrying out calibration to differentiate between data obtained using the piezoelectric sensor 1 in response to a subject coughing with a relatively small quantity of air in their lungs, and data obtained using the piezoelectric sensor 1 in response to the subject speaking, the device is able to effectively differentiate between data indicative of a subject coughing and data indicative of a subject speaking.

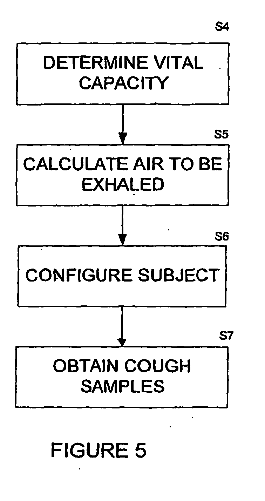

Calibration is now described with reference to Figure 5, which shows the processing of step S1 of Figure 1 in further detail. As described above, the quantity of air within a subject's lungs when calibration measurements are obtained is an important factor in the calibration process. This quantity is measured as a fraction of a subject's vital capacity, which is determined at step S4 of Figure 5. Having determined vital capacity at step S4, the quantity of air to be exhaled (following a maximum inspiration) when calibration readings are taken, is determined at step S5. At step S6, arrangements are made so as to ensure that the subject has the desired quantity of air within his/her lungs (described in further detail below). Having ensured that the necessary quantity of air is present within the subject's lungs at step S6, acoustic signals indicative of cough events are obtained at step S7 using the piezoelectric sensor 1.

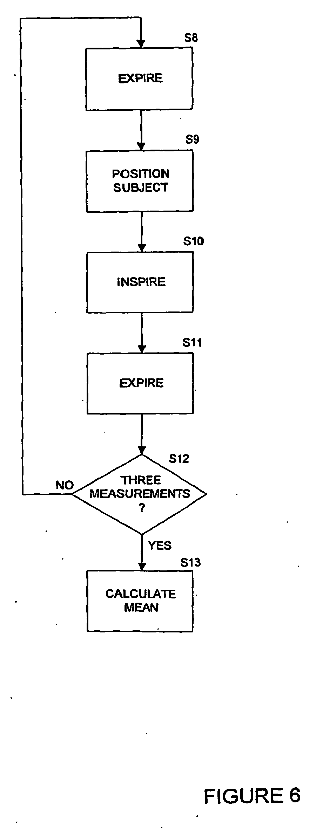

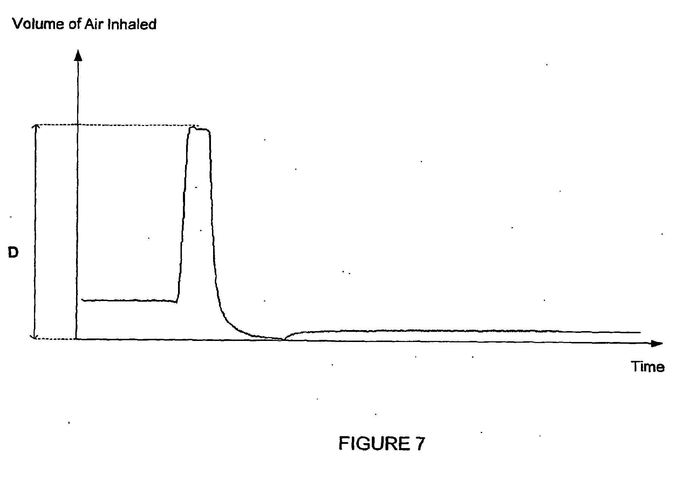

Figure 6 shows determination of vital capacity (carried out at step S4 of Figure 5) in further detail. At step S8 the subject 2 breathes out fully (a maximum expiration). At step S9, the subject is positioned such that their lips are sealed around the mouthpiece 17 of the pneumotachograph 16. Having positioned the subject in this way, the subject takes a full breath in (a maximum inspiration) at step S10, and then makes a second maximum expiration at step S11. The pneumotachograph outputs volume data which is displayed on the monitor 13 in the form of a graph as illustrated in Figure 7. The subject's vital capacity is denoted D on the graph of Figure 7. The value obtained for the vital capacity is stored in the data memory 6b of the workstation 3.

Referring back to Figure 6, step S12 ensures that steps S8 to S11 are repeated three times so as to obtain three measurements of vital capacity. Having obtained three measurements of vital capacity, the mean average of these three measurements is calculated at step S13. This average value is, for the purposes of subsequent processing, used as the vital capacity of the subject 2. In alternative embodiments of the present invention.a maximum value may be used in place of the mean value.

As explained above with reference to Figure 4, coughs with vibrations which most resemble the vibrations of speech are produced by a subject coughing with a small amount of air in his/her lungs. However, it has also been found that there is a need for a minimum quantity of air within a subject's lungs to make coughing physically possible. It is therefore currently preferred that calibration cough samples are obtained when the subject has a quantity of air that is 25%-40% of their vital capacity in their lungs. More preferably the subject has a quantity of air that is 25% to 35% of their vital capacity within their lungs, and most preferably air to 30% of the subject's vital capacity is within their lungs. In alternative embodiments of the present invention calibration cough samples are obtained when the subject has a predetermined volume of air within their lungs.

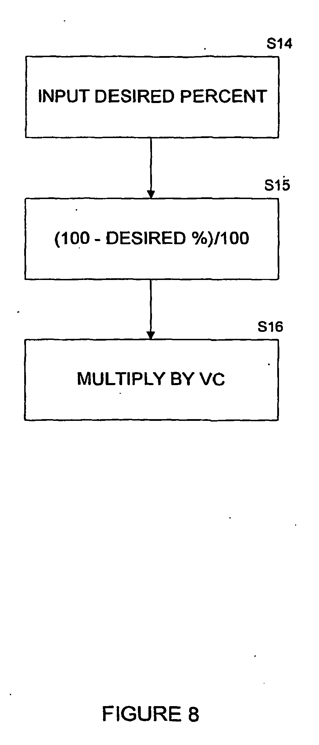

Figure 8 shows processing carried out at step S5 of Figure 5 in further detail. The desired percentage of the vital capacity is input to the workstation 3 by means of the keyboard 11 at step S 14. The input value is subtracted from 100 at step S 15, and the resulting value is then divided by 100 and the result multiplied by the vital capacity as determined at step S4 of Figure 5. Such a process can be pre-programmed into the program memory 6a. This calculation generates a value which can be used to configure the subject, prior to obtaining acoustic signals indicative of cough, as described below.

To obtain an acoustic signal indicative of a cough, the subject 2 is asked to make a maximum expiration, before sealing their lips around a mouthpiece 17 of the pneumotachograph 16. The subject then makes a maximum inspiration and breathes the calculated amount of air into the mouthpiece 17 of the pneumotachograph 16. Given the calculation illustrated in Figure 8, this will mean that the quantity of air within the subject's lungs is the percentage of the subject's vital capacity input at step S 14. The subject 2 then removes their lips from mouthpiece 17 and coughs.

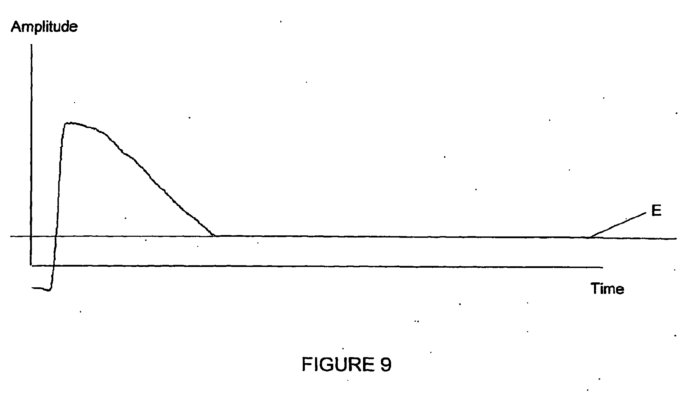

To enable the subject to become aware that they have exhaled the previously calculated amount of air, the processor 5 of the workstation 3 is programmed to provide an audible and/or visible indication, via the monitor 8 and/or the speaker 10. For example, the quantity of air and the rate at which the subject inhaled and exhaled during such a process may be displayed to the subject in the form of a graph illustrated in figure 9. The visible indication in the present embodiment takes the form of a straight line across a display on the monitor denoted E. An audible indication may take the form of a high pitched sound emitted by the speaker 10, produced when the subject has exhaled sufficiently.

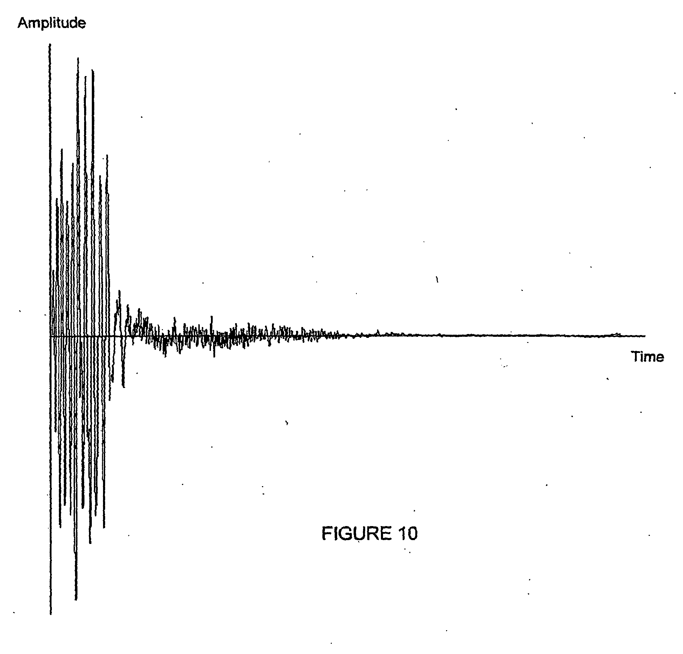

Referring back to Figure 5, steps S6 and S7 are repeated 20 times, producing 20 coughs at, preferably, 30% of the vital capacity of the subject 2. Data representative of the acoustic signals caused by the 20 coughs is recorded via the piezoelectric sensor 1, the recordings being stored on the hard disk drive 7 in the form of 20 data files. A plot of the amplitude of the vibrations against time caused by a typical cough is shown in figure 10.

In order to calibrate the apparatus used to implement the invention, it is necessary to obtain samples of the subject speaking, so as to allow the apparatus to effectively differentiate between acoustic signals attributable to cough, and acoustic signals attributable to speech. Appropriate speech samples are therefore obtained. Two sets of samples are preferably obtained, the first sample being a recording of the subject reading a specially selected passage and the second set of samples being a recording of the subject having a conventional conversation. The specially selected passage preferably contains all the phonetic sounds of the subject's native language. For a native English speaking subject, a passage known as the "The Rainbow Passage" (Fairbanks G: "Voice and Articulation Drillbook", 2nd ed., New York, Harper, 1960, pages 124-139) can be used. Alternately, the passage may just contain the phonetic sounds of a language that most resemble coughing when recorded by the piezoelectric sensor 1.

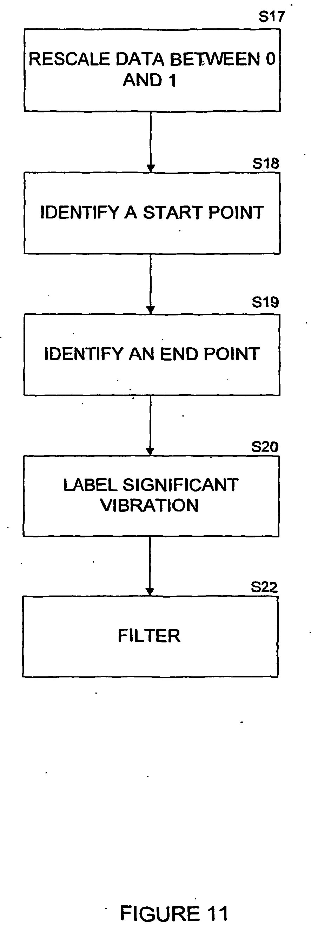

Figure 11 illustrates processing carried out on the obtained cough recordings, which are preferably sampled at a frequency of 8000Hz.The positions of individual sound events in the continuous cough and non-cough calibration data need to be identified. The speech and cough data is normalised (step 17). That is, the speech and cough data is rescaled between a maximum value of 1 and minimum value of 0. The start of a significant sound event is identified when a sample point is greater than a preset level (step 18). The point at which 400 consecutive sample points remain below a preset level indicates the end of the sound event and is identified (step 19). The positions of each event in the cough and speech calibration data are thus determined and recorded (step 20).

Analysis of the cough file will produce 20 cough sounds. The number of sound events produced by the speech calibration will be variable, depending upon the length of conversational speech and the speech manner.

Having processed each of the calibration files, each of the significant vibrations is then each filtered by a plurality of Butterworth high pass filters (step S22). Each of these filters will apply a different threshold, in the range 1.8 kHz to 4 kHz at intervals of 10Hz. The maximum amplitude within the filtered data after application of each filter is recorded. Given that 221 filters are applied, this generates a 221 point curve, each point being associated with the threshold frequency of the filter responsible for its creation. Thus, a frequency domain wave form is created. This approach has the potential for allowing real-time filtering of the source signal, and direct determination of true trigger level for cough identification in an analogue based device. Alternatively, digital sampling and standard signal processing techniques (such as Fourier Transforms) could be applied to the time domain waveform to generate the frequency domain waveform.

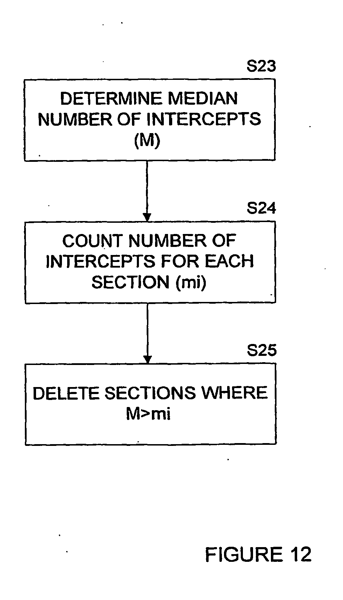

The processing represented by steps S17 to S22 of Figure 11 is repeated for each of the cough files in turn, and is also repeated for each of the speech files in turn. Thus, having repeated the processing of Figure 11 for each file, a frequency domain waveform for each obtained acoustic signal is available. The next stage of processing involves the removal of obtained sounds which are unlikely to contribute useful information to the calibration process. Such processing is carried out separately for cough samples and speech samples. The processing carried out for each type of sample is shown in Figure 12.

Referring to Figure 12, at step S23, the number of intercepts with other curves (in the frequency domain) for each curve is determined, and the median of these numbers is also determined. This generates respective median values for cough samples, M_{Cough} and speech samples. M_{Speech}.

Having generated these median values, the number of intercepts for each curve is counted, mᵢ (step S24). If a curve has a lower number of intercepts than the respective median number, then it is discarded (step S25).

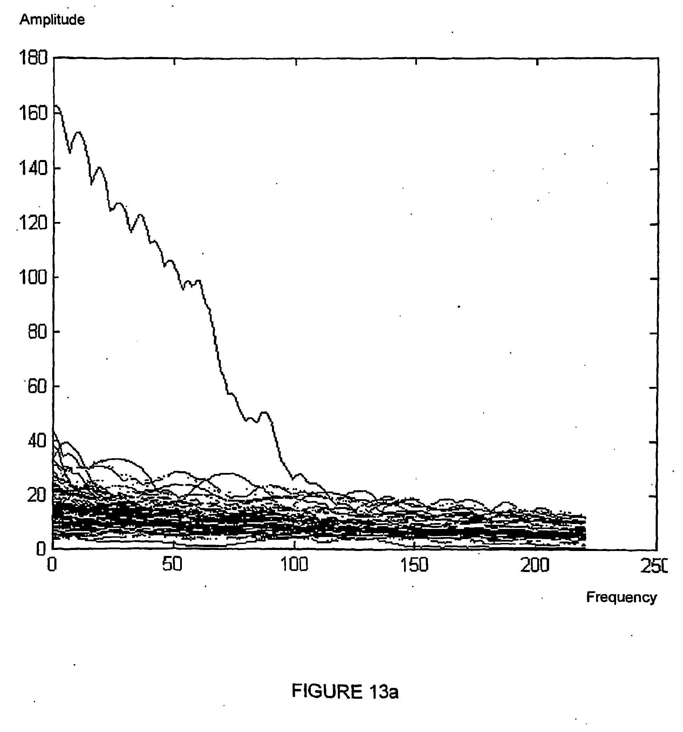

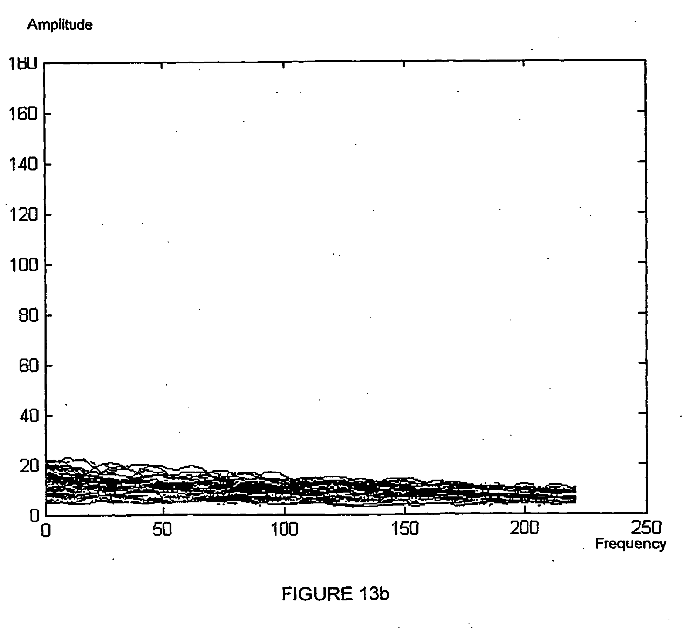

Figures 13a and 13b show the effect of removing outliers from a typical set of speech samples. Figure 13a illustrates frequency domain waveforms for each obtained speech sample, and it can be seen that one of the waveforms is clearly very different from all of the others, while others of the waveforms are considerably different to most of the others. Figure 13b shows the waveforms obtained when outlying waveforms are removed in the manner described above with reference to Figure 12.

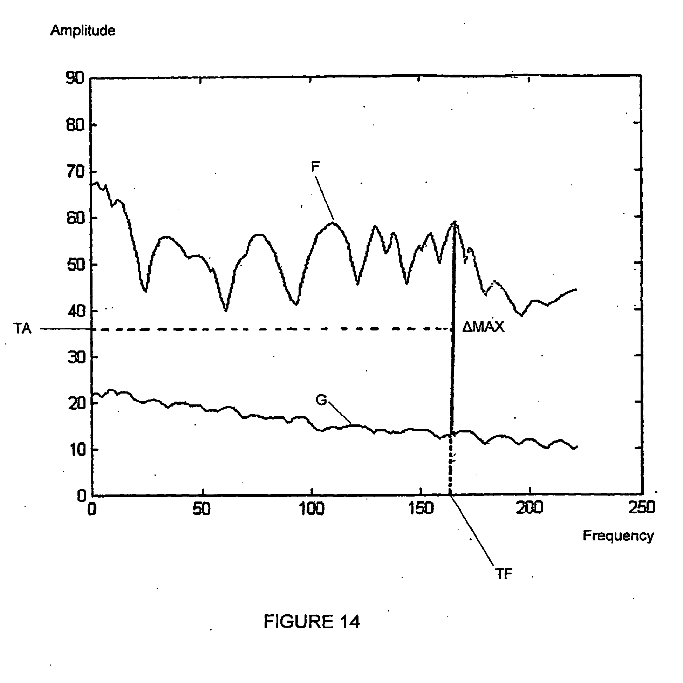

The calibration process then determines thresholds that are to be used to determine whether a received acoustic signal results from a cough or speech, Figure 14. For each frequency at which analysis is carried out, the maximum speech amplitude (over all speech calibration files) G is subtracted from the minimum cough amplitude (over all cough calibration files) F. This generates a difference value for each frequency at which analysis is carried out. The frequency at which the largest difference (ΔMAX) occurs is selected and nominated as the trigger frequency (TF) for the subject. The frequencies at which the second, third, fourth and fifth largest differences occur are selected and nominated as the confirmatory frequencies (CF1, CF2, CF3, CF4) for the subject.

The midpoint between the cough point and the speech point at the trigger frequency defines the trigger amplitude (TA). Subsequently, if during the monitoring period the amplitude of a noise at the trigger frequency (TF) exceeds this trigger amplitude (TA), it will be designated a potential cough, and if the amplitude at the trigger frequency (TF), is less than the trigger amplitude (TA) it will be designated a non-cough event.

Similarly, the midpoint between the cough points and the speech points at the confirmatory frequencies (CF1, CF2, CF3, CF4) define four confirmatory amplitudes (CA1, CA2, CA3, CA4). The confirmatory amplitudes (CA1, CA2, CA3, CA4) are used to help assess whether a sound was a cough or speech.

Referring back to Figure 1, the description presented above, has been concerned with calibration carried out at step S1. Operation of step S2 is now described.

Having completed calibration as described above, the sensor 1 remains affixed to the subject in the manner described above for a predetermined time period. The sensor interface 4 also accompanies the subject during this time, and can conveniently be provided with a belt clip or the like to ease transportation. The sensor interface 4 makes a continuous recording of data obtained by the sensor 1 in the non-volatile storage 19, under control of the processor 18. In addition to the piezoelectric sensor described above, an air-coupled microphone is also positioned on the lapel of the patient in preferred embodiments of the invention. The lapel microphone records all noises that are made during the period the subject is wearing the sensor. The microphone recording can be used to validate the results of the piezoelectric sensor manually or automatically and can be used as a diagnostic tool e.g. for detecting sputum or wheeze in a cough.

Data obtained by the sensor interface 4 is then provided to the workstation 3 for analysis. This data provision can be by means of direct a wired connection over, for example a serial interface or memory card transfer. However in alternative embodiments of the invention data is transferred from the sensor interface 4 to the workstation 3 over a computer network. For example, a user may connect the sensor interface 4 to a modem and telephone line to allow data to be transmitted to the workstation 3. Alternatively, the sensor interface 4 may include telecommunications means allowing data to be passed to the workstation 3 in real time.

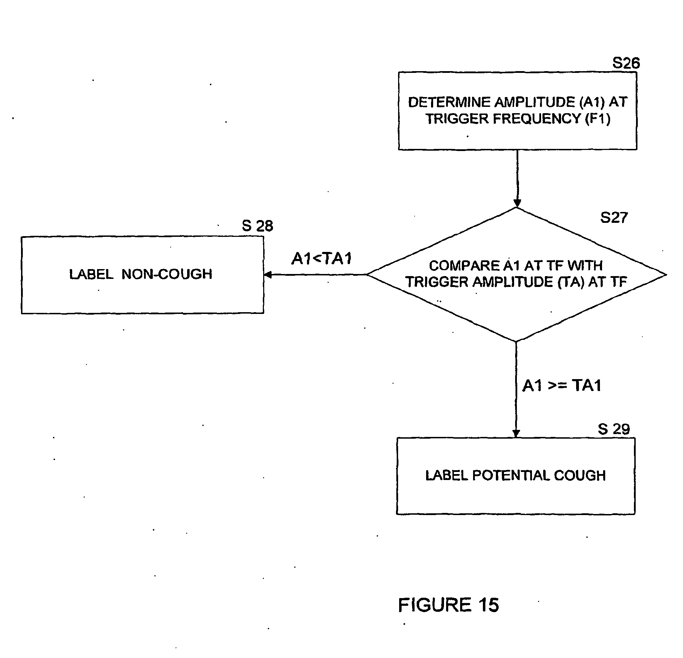

Data received at the workstation 3 is processed at step S3 of Figure 1. This processing involves identification of significant vibrations as described above. Each significant vibration is filtered as described above. Having carried out this filtering, an amplitude for each frequency value of interest within a particular significant vibration is obtained. This data is then analysed to determine the number of coughs made by the subject in the period. The analysis is divided into two stages. A first stage is shown in the flowchart of Figure 15.

Referring to Figure 15, at step S26, the amplitude of each significant vibration at the trigger frequency (TF) is determined. The determined amplitude is compared with the trigger amplitude (step S27). If the determined amplitude does not exceed the trigger amplitude (TA), the recording is rejected and labelled a non-cough at step S28. If that amplitude exceeds the trigger amplitude (TA), the recording of the associated vibration is labelled a potential cough at step S29 and output to a second stage of analysis.

In the second stage of analysis, the potential cough is analysed at each of the confirmatory frequencies (CF). The amplitude of the vibrations at these frequencies is compared to the corresponding confirmatory amplitudes (CA). If the amplitudes of vibrations caused by the processed signal, at the confirmatory frequencies, are larger than:
1. all four confirmatory amplitudes at the respective confirmatory frequencies, then the noise is determined to be a cough and appropriate data is recorded;
2. only three confirmatory amplitudes at the respective confirmatory frequencies, then the noise is recorded as a potential cough implying a manual inspection using data obtained using the lapel microphone to be necessary; and
3. only two, one or none of the confirmatory amplitudes at the respective confirmatory frequencies, then the noise is recorded as a non-cough.

It has been described above how acoustic signals relating to cough events can be distinguished from acoustic signals relating to non-cough events, by comparing the amplitude of an acoustic signal at a range of predetermined frequency components against primary and secondary thresholds. A method of determining the predetermined frequency components, and the respective primary and secondary thresholds has also been described.

The above-described embodiments of the present invention make no distinction between a cough event for which a patient coughs at a low percentage of their vital capacity and a cough event for which a patient coughs at a high percentage of their vital capacity. Both types of cough event are treated the same for the purposes of measuring the rate at which a patient coughs. However, the percentage of vital capacity (or alternatively the absolute lung volume) from which a patient coughs can provide important clinical information to a health care professional. Certain embodiments of the present invention provide clinical information relating to the vital capacity, or absolute lung volume, at which a patient coughs. That is, for an acoustic signal known to relate to a cough event, the vital capacity of the patient from which they coughed can be determined.

The present inventors have realised that the percentage of vital capacity at which a patient coughs determines characteristics of a cough sound recorded using a chest monitor as shown in Figure 2. The relationship between the percentage of vital capacity held in a patient's lungs and the cough acoustic signal recorded via a contact microphone placed over the trachea has been investigated. It has been found that the intensity of the acoustic signal (that is the amplitude of the acoustic signal) for a cough event recorded at the trachea is proportional to the air flowing from the patient's lungs. This relationship is particularly strong for frequency components of the acoustic signal above 1 kHz.

Acoustic signal amplitude can be plotted against time, for an acoustic signal relating to a cough event. It follows from the correlation of acoustic signal amplitude and airflow that the integral of the acoustic signal (that is the area under the amplitude/time graph) is proportional to the volume of air expelled from the patient's lungs, regardless of the instantaneous or peak cough intensity.

For the purposes of the present invention it is assumed that the volume of air expelled during a cough event is proportional to the volume of air inspired prior to the cough. It has been shown that for high pass filtered acoustic signals relating to cough events at a particular cut off frequency for that patient, there is a good relationship between the volume inspired prior to the cough and the volume expired during the cough, and thus the integral of the acoustic signal. It is possible that at high inspired volumes (for instance, over 70% of a patients vital capacity), it is not possible for the patient to expire all of the inspired air during a single cough. It has been observed that some patients have difficulty in voluntarily coughing from such higher volumes. Such patients may also not spontaneously cough from such high volumes, reducing the significance of patients not expiring all of their lung volume during a cough event at a high percentage of vital capacity.

As discussed above, the peak amplitude of an acoustic signal relating to a cough event is not indicative of the volume of air inspired prior to the cough event (the percentage of vital capacity at which the patient coughs). However, for a high pass filtered acoustic signal (with a cut of frequency over 1kHz), the peak amplitude of the signal is related to the maximum airflow from the lungs during the cough event. The integral of the filtered acoustic signal is thus related to the percentage of vital capacity for the patient immediately prior to the cough event.

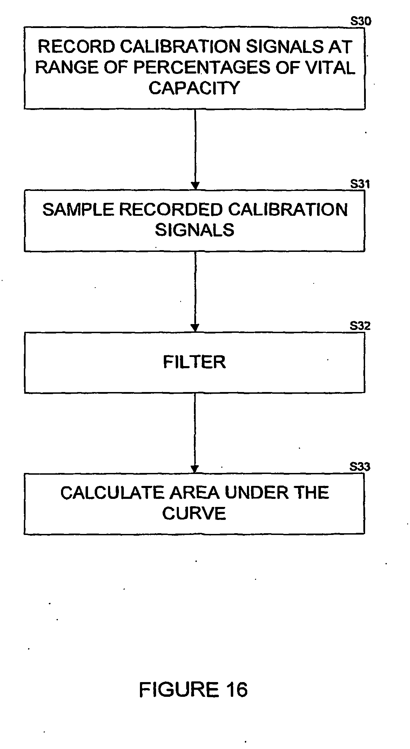

In order to determine the percentage of vital capacity at which a patient coughs it is first necessary to calibrate the apparatus. The process of capturing calibration acoustic signals at a range of percentages of vital capacity is the same as described above. Referring now to Figure 16, for a given subject, three calibration cough events are recorded at each of a range of volumes (step 30). The range of volumes are between 10% and 90% of the subject's vital capacity in 10% steps (giving a total of 27 recorded cough events). Each cough event is sampled from the recorded acoustic signals by identifying the start and finish points of the cough event, in the same manner as discussed above.

Each recorded cough event is sampled at a sampling frequency of 8 kHz (step 31). Each cough event is then successively filtered using a high pass filter, having a cut-off threshold varying between 1.4 kHz and 3.6 kHz, in steps of 200 Hz (step 32). The frequency range is chosen to minimise the effect of any speech components within the recorded cough events.

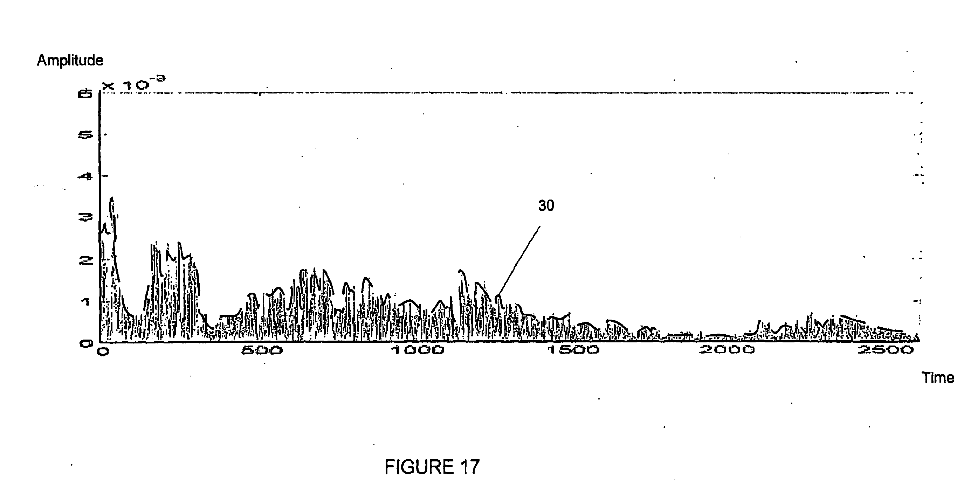

At step 33 the area under the curve is calculated for each differently filtered acoustic signal, for each of the 2 cough events. The area under the curve is equal to the integral of the amplitude / time plot over the duration of a cough event. Referring now to Figure 17, this illustrates the area under the curve for an acoustic signal relating to a cough event. The area under the curve is approximately bordered by dashed line 30.

In certain embodiments of the present invention a determination is made of the integral of an acoustic signal (i.e. the area under an amplitude / time plot over the duration of a cough event. It will be appreciated that the phrase "determining the integral" is not limited to a calculation of the true integral of a signal. There are many types of numerical analysis that can be performed on a signal to give an indication of the area under a curve. These approaches do not necessarily give an accurate measure of the true integral, however, for the purposes of the present invention they may be sufficient. "Determining the integral" is intended to cover such numerical analysis.

The above described processing provides a calibration dataset for cough events, giving a value for the area under the amplitude / time curve for three sampled cough events at a range of percentages of vital capacity (between 10% and 90%). The calibration dataset is then processed to determine a patient specific threshold to be applied to acoustic signals of cough events to determine the lung volume from which the patient coughed.

For each high pass filter threshold, there are three area under the curve values for each percentage of vital capacity of lung volume. These may be plotted against lung volume, for each filter threshold level. Linear regression is then applied to the calibration dataset, effectively establishing which filter threshold produces the most linear response. That is, the plotted data for each of the filter levels is processed to determine which filter level gives the closest to a straight line linear relationship, when the area under the curve (integral) is plotted against lung volume. This can alternatively be considered a process of comparing each dataset (each filter threshold) with an operating dataset comprising a straight line plot of area under the curve for an acoustic signal against lung volume, and determining which dataset gives the closest fit.

A subject, after inspiring a volume of air prior to coughing may expire this volume quickly with maximum intensity (amplitude) or they may expire the volume of air slowly with a reduced intensity (amplitude). Calculating the area under the curve gives a better estimation of true lung volume inspired prior to the cough than any estimate based solely on the peak amplitude of the cough event. The peak amplitude of a filtered acoustic signal representing a cough event is a measure only of intensity.

After processing, the chosen high pass filter threshold can be applied to acoustic signals representing new involuntary cough events in order to gauge the volume of air within a subjects lungs when they coughed. As described above, for a given subject, for which the apparatus has been calibrated, a determination as to whether a recorded acoustic signal relates to a cough event can be made by determining whether certain frequency components exceed predetermined thresholds. Alternatively, the determination as to whether an acoustic event relates to a cough event can be made in some other way, for instance a health care professional can manually determine whether a recorded acoustic signal relates to a cough event.

If it is determined that the acoustic signal does relate to a cough event, then the percentage of vital capacity, or absolute lung volume, at which the patient coughed can be determined, by reference to the calibration data. Firstly, the new acoustic signal is high pass filtered applying the filter threshold determined from the linear regression on the calibration dataset. Secondly, the area under the acoustic amplitude / time curve for the recorded cough event is calculated. The calculated area under the curve value is then compared to the plot area under curve against lung volume generated by the linear regression for that filter threshold. From this, an estimate of the lung volume from which the patient coughed can be calculated.

In accordance with further embodiments of the present invention, calibration data relating to the area under an amplitude/time graph can also be used directly to make a determination as to whether a recorded acoustic event relates to a cough or a non-cough. Cough sounds measured using the sensor depicted in Figure 2 contain a greater proportion of higher frequency components than most speech sounds. Furthermore, high frequency components that occur during speech tend to be short lived, and thus do no contribute greatly to the area under the curve calculation for an acoustic signal representing speech. For the method of differentiating between cough and non-cough events described in relation to Figures 1 to 14, a very short lived, but high frequency component in speech could falsely indicate the presence of speech.

Conversely, by high pass filtering an acoustic signal the lower frequency components characterised by speech are removed. By applying a cough/non-cough differentiation technique in accordance with a further embodiment of the present invention based upon calculating the area under the curve for a cough event, improved differentiation between cough events and non-cough events is made possible.

The basis of this alternative method of distinguishing between cough and non-cough events is processing data sets comprising for a given subject cough and non-cough events (such as speech) in the same way as described above in connection with Figures 16 and 17. For both types of acoustic signal, the acoustic signal is high pass filtered at a range of cut of thresholds. For each filtered signal, the area under the curve (i.e. the integral of the acoustic signal) is determined. The area under the curve values for cough and non-cough events are then compared, to determine the high pass filter threshold at which there is on average the greatest difference between the area under the curve values.

This calculated frequency threshold (which may not be the same as threshold as described above in relation to determining the lung volume from which a patient coughs) can then be used to determine whether a new acoustic signal relates to a cough or a non-cough event. A new acoustic signal is firstly high pass filtered using the calculated threshold. The filtered signal is then process is determine the area under the curve value.

The area under the curve value for the new acoustic signal is then compared to a threshold value. If the calculated value exceeds the threshold then it is determined that the new acoustic signal relates to a cough event. If the calculated value is below the threshold then it is determined that the new acoustic signal relates to a non-cough event. One method of determining the area under the curve threshold is to set the threshold half way between the average area under the curve value for cough events and the average area under the curve value for non-cough events, when both are high pass filtered at that cut-off frequency.

Although preferred embodiments of the present invention have been described in detail, it will be appreciated that other implementations arc possible without departing from the scope of the present invention, as set out in the appended claims. In particular, where references have been made in the forgoing to piezo electric sensors, it will be appreciated that other suitable sensors can similarly be used.

## Claims

1. A calibration method for calibrating an apparatus to differentiate acoustic signals attributable to cough from other acoustic signals, the method comprising:
receiving first data representing a first acoustic signal representing at least one cough;
receiving second data representing a second acoustic signal representing an acoustic event other than a cough; and
processing said first and second data to generate configuration data for said apparatus, said configuration data defining at least one rule which when applied differentiates acoustic signals attributable to cough from other acoustic signals.

2. A method according to claim 1, further comprising:
filtering at least one of said first and second data to remove vibrations below a predetermined amplitude.

3. A method according to claim 1 or 2, further comprising:
filtering the received data representing at least one cough by removing data that represents vibrations with a frequency that exceeds an upper cough cut off frequency.

4. A method according to any preceding claim, further comprising:
filtering at least one of said first and second data to remove frequency components above a predetermined frequency threshold.

5. A method according to any preceding claim, further comprising:
filtering at least one of said first and second data to remove frequency components below a predetermined frequency threshold.

6. A method according to any preceding claim, wherein processing said first and second data to generate configuration data comprises:
determining the amplitude of a plurality of predetermined frequency components of each of said first and second acoustic signals.

7. A method according to any preceding claim, wherein said rule is defined by at least one selected frequency, the or each selected frequency having an associated amplitude.

8. A method according to claim 7, wherein the or each selected frequency is selected from said plurality of predetermined frequencies such that the amplitude of said first and second acoustic signals at said selected frequencies has a difference greater than the difference between the amplitude of said first and second acoustic signals at all others of said predetermined frequencies.

9. A method according to claim 8, wherein the amplitude associated with each selected frequency is defined to be centrally placed between the amplitude of said first acoustic signal, and said amplitude of said second acoustic signal at the respective selected frequency.

10. A carrier medium carrying computer program code means to cause a computer to carry out a method according to any one of claims 1 to 9.

11. A computer apparatus comprising:
a program memory containing processor readable instructions; and
a processor for reading and executing the instructions contained in the program memory;
wherein said processor readable instructions comprise instructions controlling the processor to carry out the method of any one of claims 1 to 10.

## Patentansprüche

1. Kalibrierverfähren zum Kalibrieren einer Vorrichtung, um akustische Signale, die Husten zurechenbar sind, von anderen akustischen Signalen zu unterscheiden, wobei das Verfahren umfasst:
Empfangen erster Daten, die ein erstes akustisches Signal darstellen, das mindestens ein Husten darstellt;
Empfangen zweiter Daten, die ein zweites akustisches Signal darstellen, das ein anderes akustisches Ereignis als ein Husten darstellt; und
Verarbeiten der ersten und zweiten Daten, um Konfigurationsdaten für die Vorrichtung zu erzeugen, wobei die Konfigurationsdaten mindestens eine Regel definieren, die bei Anwendung akustische Signale, die Husten zurechenbar sind, von anderen akustischen Signalen unterscheidet.

2. Verfahren nach Anspruch 1, ferner umfassend:
Filtern mindestens der ersten und/oder zweiten Daten, um Vibrationen unterhalb einer vorbestimmten Amplitude zu entfernen.

3. Verfahren nach Anspruch 1 oder 2, ferner umfassend:
Filtern der empfangenen Daten, die mindestens ein Husten darstellen, durch Entfernen von Daten, die Vibrationen mit einer Frequenz, die eine obere Hustenbegrenzungsfrequenz überschreitet, darstellen.

4. Verfahren nach einem der vorhergehenden Ansprüche, ferner umfassend:
Filtern mindestens der ersten und/oder zweiten Daten, um Frequenzkomponenten oberhalb eines vorbestimmten Frequenzschwellwerts zu entfernen.

5. Verfahren nach einem der vorhergehenden Ansprüche, ferner umfassend:
Filtern mindestens der ersten und/oder zweiten Daten, um Frequenzkomponenten unterhalb eines vorbestimmten Frequenzschwellwerts zu entfernen.

6. Verfahren nach einem der vorhergehenden Ansprüche, wobei das Verarbeiten der ersten und zweiten Daten, um Konfigurationsdaten zu erzeugen, umfasst:
Bestimmen der Amplitude einer Vielzahl von vorbestimmten Frequenzkomponenten jedes der ersten und zweiten akustischen Signale.

7. Verfahren nach einem der vorhergehenden Ansprüche, wobei die Regel durch mindestens eine ausgewählte Frequenz definiert wird, wobei die oder jede ausgewählte Frequenz eine zugeordnete Amplitude aufweist.

8. Verfahren nach Anspruch 7, wobei die oder jede ausgewählte Frequenz so aus der Vielzahl von vorbestimmten Frequenzen ausgewählt wird, dass die Amplitude der ersten und zweiten akustischen Signale bei den ausgewählten Frequenzen eine Differenz aufweist, die größer als die Differenz zwischen den Amplituden der ersten und zweiten akustischen Signale bei allen anderen der vorbestimmten Frequenzen ist.

9. Verfahren nach Anspruch 8, wobei die jeder ausgewählten Frequenz zugeordnete Amplitude so definiert wird, dass sie in der Mitte zwischen der Amplitude des ersten akustischen Signals und der Amplitude des zweiten akustischen Signals bei der jcweiligen ausgewählten Frequenz liegt.

10. Trägermedium, das Computerprogrammcodemittel trägt, um einen Computer zu veranlassen, ein Verfahren nach einem der Ansprüche 1 bis 9 auszuführen.

11. Computervorrichtung, umfassend:
einen Programmspeicher, der prozessorlesbare Anweisungen enthält; und
einen Prozessor zum Lesen und Ausführen der im Programmspeicher enthaltenen Anweisungen;
wobei die prozessorlesbaren Anweisungen Anweisungen umfassen, die den Prozessor steuern, um das Verfahren nach einem der Ansprüche 1 bis 10 auszuführen.

## Revendications

1. Procédé d'étalonnage destiné à étalonner un dispositif en vue de différencier des signaux acoustiques imputables à de la toux relativement à d'autres signaux acoustiques, le procédé comprenant les étapes ci-dessous consistant à :
recevoir des premières données représentant un premier signal acoustique représentant au moins une toux ;
recevoir des secondes données représentant un second signal acoustique représentant un événement acoustique autre que de la toux ; et
traiter lesdites premières et secondes données afin de générer des données de configuration pour ledit dispositif, lesdites données de configuration définissant au moins une règle laquelle, lorsqu'elle est appliquée, différencie des signaux acoustiques attribuables à de la toux relativement à d'autres signaux acoustiques.

2. Procédé selon la revendication 1, comprenant en outre l'étape ci-dessous consistant à :
filtrer au moins l'une desdites premières et secondes données pour éliminer les vibrations en dessous d'une amplitude prédéterminée.

3. Procédé selon la revendication 1 ou 2, comprenant en outre l'étape ci-dessous consistant à :
filtrer les données reçues représentant au moins une toux en supprimant des données qui représentent des vibrations dont une fréquence dépasse une fréquence de coupure de toux supérieure.

4. Procédé selon l'une quelconque des revendications précédentes, comprenant en outre l'étape ci-dessous consistant à :
filtrer au moins l'une desdites premières et secondes données en vue d'éliminer des composantes de fréquence supérieures à un seuil de fréquence prédéterminé.

5. Procédé selon l'une quelconque des revendications précédentes, comprenant en outre l'étape ci-dessous consistant à :
filtrer au moins l'une desdites premières et secondes données en vue d'éliminer des composantes de fréquence inférieures à un seuil de fréquence prédéterminé.

6. Procédé selon l'une quelconque des revendications précédentes, dans lequel l'étape consistant à traiter lesdites premières et secondes données pour générer des données de configuration comprend l'étape ci-dessous consistant à :
déterminer l'amplitude d'une pluralité de composantes de fréquence prédéterminées de chacun desdits premier et second signaux acoustiques.

7. Procédé selon l'une quelconque des revendications précédentes, dans lequel ladite règle est définie par au moins une fréquence sélectionnée, ladite une ou chaque fréquence sélectionnée présentant une amplitude associée.

8. Procédé selon la revendication 7, dans lequel ladite une ou chaque fréquence sélectionnée est sélectionnée parmi ladite pluralité de fréquences prédéterminées de sorte que l'amplitude desdits premier et second signaux acoustiques auxdites fréquences sélectionnées présente une différence supérieure à la différence entre l'amplitude desdits premier et second signaux acoustiques à toutes les autres desdites fréquences prédéterminées.

9. Procédé selon la revendication 8, dans lequel l'amplitude associée à chaque fréquence sélectionnée est définie comme étant placée centralement entre l'amplitude dudit premier signal acoustique et ladite amplitude dudit second signal acoustique à la fréquence sélectionnée respective.

10. Support informatique contenant un moyen de code de programme informatique en vue d'amener un ordinateur à mettre en oeuvre le procédé selon l'une quelconque des revendications 1 à 9.

11. Dispositif informatique comprenant :
une mémoire de programme contenant des instructions lisibles par un professeur et
un processeur pour lire et exécuter les instructions contenues dans la mémoire de programme ;
où lesdites instructions lisibles par un processeur incluent des instructions amenant le processeur à mettre en oeuvre le procédé selon l'une quelconque des revendications 1 à 10.
